## Europäisches Patentamt
## European Patent Office
## Office européen des brevets

(19)

(11) Veröffentlichungsnummer: **0 156 133**
**B1**

(12) # EUROPÄISCHE PATENTSCHRIFT

(45) Veröffentlichungstag der Patentschrift:
14.01.87

(51) Int. Cl.⁴: **C 07 C 67/08,** C 07 C 69/82

(21) Anmeldenummer: **85101275.7**

(22) Anmeldetag: **07.02.85**

(54) Verfahren zur Herstellung von Terephthalsäuredimethylester aus p-Xylol und Methanol.

(30) Priorität: **03.03.84 DE 3407925**

(43) Veröffentlichungstag der Anmeldung:
**02.10.85 Patentblatt 85/40**

(45) Bekanntmachung des Hinweises auf die Patenterteilung:
**14.01.87 Patentblatt 87/3**

(84) Benannte Vertragsstaaten:
**BE DE FR GB IT NL**

(56) Entgegenhaltungen:
GB-A-878 063
US-A-3 076 019

(73) Patentinhaber: **DYNAMIT NOBEL AKTIENGESELLSCHAFT, Postfach 1261, D-5210 Troisdorf, Bez. Köln (DE)**

(72) Erfinder: **Modic, Rudolf, Dr., Waldfrieden 8, D-3074 Steyerberg (DE)**
Erfinder: **Porschen, Jörg, Merzenicher Strasse 154, D-5160 Düren (DE)**
Erfinder: **Schoengen, Anton, In den Espeln 9, D-5810 Witten (DE)**
Erfinder: **Wirges, Ralf, Porzer Strasse 36, D-5216 Niederkassel (DE)**

EP 0 156 133 B1

## Beschreibung

Die Erfindung betrifft ein Verfahren zur Herstellung von Terephthalsäuredimethylester aus p-Xylol und Methanol der im Oberbegriff von Patentanspruch 1 bezeichneten Art.

Bei diesem industriell in großem Umfang ausgeübten Verfahren (vgl. Hydrocarbon Processing, November 1983, Seite 91) wird in der Veresterung das aus der Oxidation kommende Oxidat mit Methanol zu Rohester verestert. Das Gewichtsverhältnis von Methanol zu Oxidat beträgt dabei zwischen etwa 0,2 und 1,0. Von der bei großtechnischer Durchführung in die Veresterung gefahrenen Methanolmenge werden nur etwa 30 bis 50 % für die Veresterung benötigt. Das restliche Methanol dient dazu, das Veresterungsgleichgewicht in Richtung einer möglichst vollständigen Veresterung des Oxidats zu verschieben, des weiteren als Energieträger und als Trägermedium für das bei der Veresterungsreaktion entstehende Wasser. Der in die Veresterung eingesetzte Methanoldampf wird um etwa 0 bis 50° C über Veresterungstemperatur überhitzt in die Veresterung eingespeist.

Bei der bisher ausgeübten Arbeitsweise wird das Filtrat der Umkristallisation von Roh-DMT in Methanol in einer Eindampfung bei etwa 65 bis 70° C mit Niederdruckdampf von etwa 1,0 bis 1,8 bar eingedampft. Die entstehenden Brüden werden kondensiert und in den Vorratstank für Reaktionsmethanol gepumpt. Das Sumpfprodukt der Filtrateindampfung wird zur weiteren Aufarbeitung in das Verfahren zurückgeführt. Das Veresterungsmethanol wird dem Behälter für Reaktionsmethanol entnommen, über Hochdruckpumpen auf beispielsweise einen Druck von 27 bar gebracht, bei diesem Druck aufgeheizt und verdampft, anschließend auf beispielsweise etwa 250 bis 270° C überhitzt und der Veresterung zugeführt. Analog wird das für die evtl. reaktive Behandlung mit Methanol einer Rückstandsfraktion der destillativen Aufarbeitung des Rohesters benötigte Methanol auf erhöhten Druck, beispielsweise 3 bis 6 bar, und eine Überhitzungstemperatur von beispielsweise 270° C gebracht. Das Überschußmethanol der Veresterung wird zusammen mit dem darin enthaltenen Reaktionswasser nach Entspannung einer Rektifikation unterworfen. Die Kopffraktion wird mit Kühlwasser kondensiert und als Flüssigkeit dem Prozeß wieder zugeführt.

Der Nachteil dieser Verfahrensweise ist, daß das Methanol für die Veresterung und die evtl. reaktive Rückstandsbehandlung somit zweimal verdampft wird.

Es bestand ein erhebliches wirtschaftliches Interesse an einem neuen System der Reaktionsmethanolversorgung in großtechnischen Anlagen zur Herstellung von DMT nach dem Verfahren der eingangs angegebenen Art, welches energiemäßig güstiger arbeitet als das mit einer zweimaligen Methanolverdampfung arbeitende bekannte System.

Diese Aufgabe wird bei einem Verfahren der eingangs angegebenen Art dadurch gelöst, daß die Merkmale des Kennzeichens von Patentanspruch 1 verwirklicht sind.

Das vorgeschlagene System der Versorgung der Veresterung mit Reaktionsmethanol auf dem benötigten Druck-und Temperaturniveau wird so durchgeführt, daß das Reaktionsmethanol durch Eindampfung oder auch Rektifikation eines methanol-haltigen Prozenstromes unter einem Druck, der unterhalb des Veresterungsdruckes liegt, gewonnen wird und daß die Methanolbrüden anschließend auf den bei der Veresterung angewandten Druck komprimiert werden, wobei die Kompressionswärme zur Temperaturerhöhung des Methanoldampfes auf die bei der Veresterung benötigte Temperatur ausgenutzt wird. Der zur Veresterung eingesetzte methanol-haltige Dampf wird im allgemeinen durch Eindampfung oder auch Rektifikation bei einem Druck von 2 bis 20 vorzugsweise 4 bis 8 bar, erzeugt. Das Verdichtungsverhältnis des der Kompression unterworfenen methanol-haltigen Dampfes wird im allgemeinen auf einen Wert zwischen 1,2 : 1 und 15 : 1, vorzugsweise 3 : 1 und 9 : 1, eingestellt, wobei die bei der Kompression erreichten Endtemperaturen des den Verdichter verlassenden Reaktionsmethanols bei einer Temperatur zwischen 150 und 300, vorzugsweise 220 bis 280° C, liegen.

Die Energie der aus der Veresterung am Kopf der Veresterungskolonne austretenden Brüden aus Überschußmethanol und Reaktionswasser kann vorteilhaft nach einer Waschung mit Prozenwasser oder auch dephlegmiertem Rücklauf der Veresterungskolonne durch Entspannung in einer Turbine genutzt werden.

Für die Eindampfung oder auch Rektifikation kann mit Vorteil die in der Oxidation freiwerdende Reaktionswärme in Form des im Kühlsystem der Oxidatoren anfallenden Niederdruckdampfes genutzt werden.

Eine besonders vorteilhafte Kombination der wesentlichen Merkmale des vorgeschlagenen neuen Systems der Zurverfügungstellung des Reaktionsmethanols für die Veresterung besteht in Folgendem:

1. Das Filtrat der Umkristallisation der Roh-DMT-Fraktion in Methanol oder auch die methanol-haltigen Veresterungsbrüden und Prozeßströme werden mit dem im Kühlsystem der Oxidation einer grontechnischen DMT-Anlage anfallenden Niederdruckdampf bei einem Druck von vorzugsweise 4 bis 8 bar eingedampft oder auch rektifiziert.

2. Der mit einem Druck von vorzugsweise 4 bis 8 bar anfallende Methanoldampf wird in einem Methanolkompressor auf einen Druck von vorzugsweise 25 bis 30 bar komprimiert und der Veresterung des Oxidats zugeführt. Die notwendige Uberhitzungstemperatur des Reaktionsmethanols für die Veresterung wird durch den Temperaturanstieg bei der Verdichtung ganz oder teilweise erreicht.

3. Ein Teil des nach 1. mit einem Druck von 4 bis 8 bar anfallenden Methanoldampfes kann - nach entsprechender Überhitzung - als Reaktionsmethanol einer Verfahrensstufe der reaktiven Behandlung einer Rückstandsfraktion zugeführt werden.

Der Haupteffekt bei dem vorgeschlagenen neuen Verfahren besteht darin, daß wertvolle Primärenergie, die in Form von hochgespanntem Dampf (17 bis 25 bar) oder Wärmeträgern zur Aufheizung, Verdampfung und Überhitzung des Veresterungsmethanols auf Reaktionstemperatur benötigt würde, eingespart werden kann.

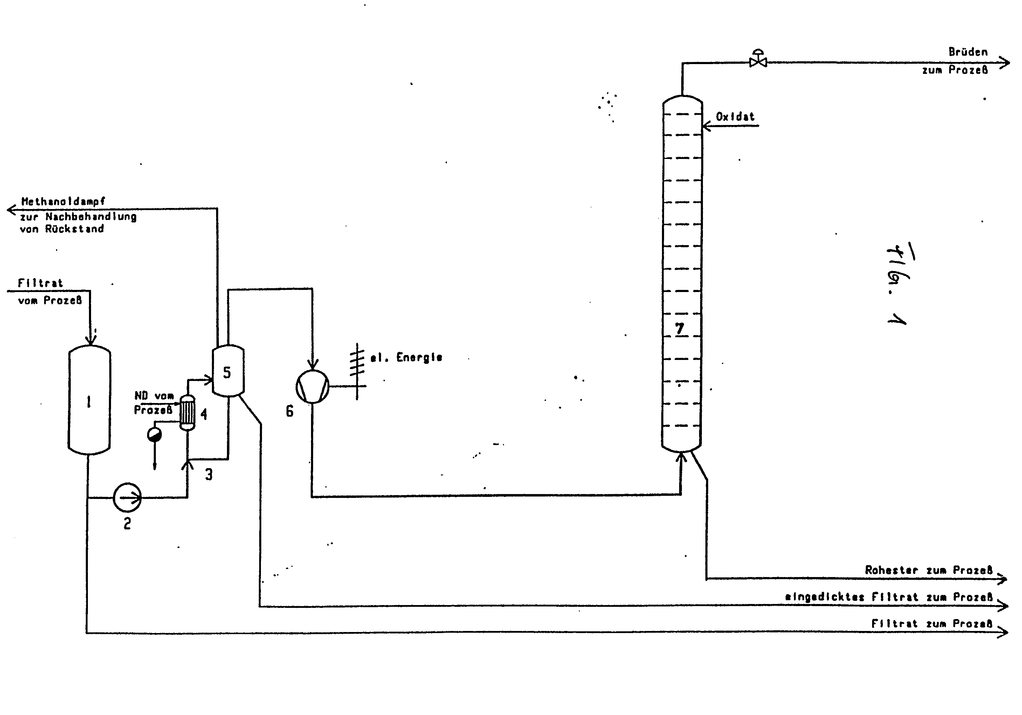

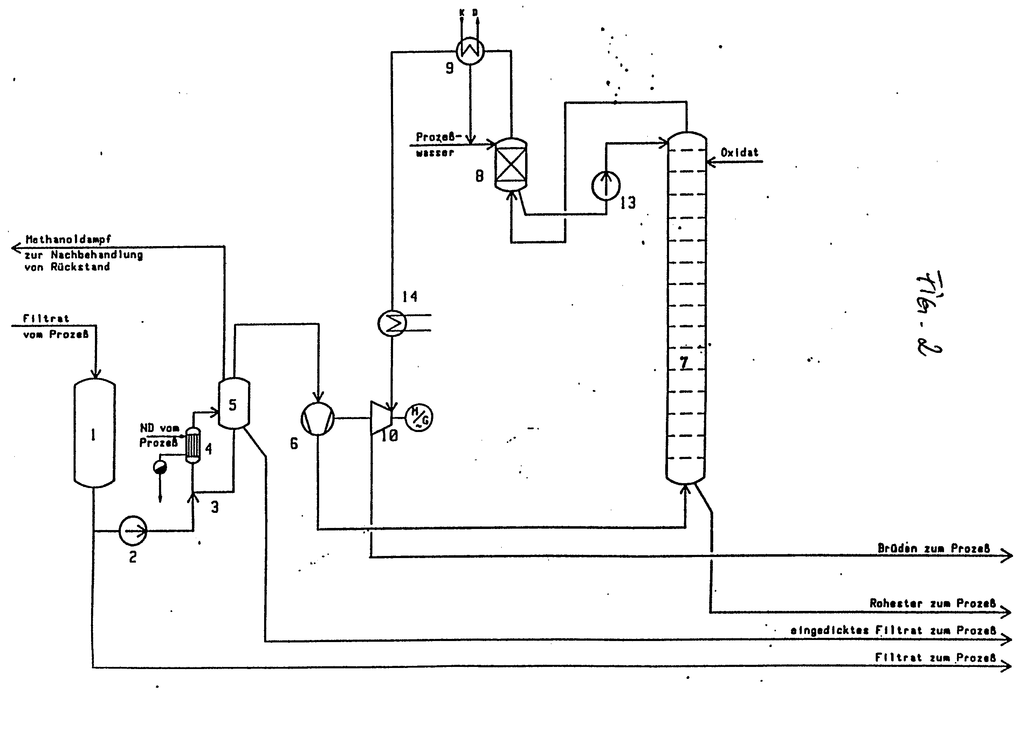

Zwei bevorzugte Verfahrensvarianten des erfindungsgemänen Verfahrens sind nachfolgend anhand der Figuren 1 und 2 der Zeichnung weiter erläutert.

Das Filtrat der Umkristallisation von Roh-DMT in Methanol wird aus dem Behälter 1 mit der Pumpe 2 in die Umwälzleitung 3 eingespeist. Filtratmengen, die nicht zur Reaktionsmethanolversorgung benötigt werden, können in das Verfahren zurückgeführt werden. Im Umlaufverdampfer 4 wird das Reaktionsmethanol über den Ausdampfbehälter 5 unter einem Druck von etwa 4 bis 8 bar ausgedampft. Der Umlaufverdampfer wird mit an anderer Stelle des Verfahrens anfallendem Niederdruckdampf (ND) beheizt. Ein Teil des Reaktionsmethanols kann für die reaktive Nachbehandlung von Bückstandsfraktionen aus der destillativen Aufarbeitung des kohesters einer Rückstandsnachbehandlung zugeführt werden. Das für die Veresterung bestimmte Reaktionsmethanol wird am Kopf des Ausdampfbehälters 5 dampfförmig abgezogen. Das Reaktionsmethanol wird in dem Methanolkompressor 6 auf etwa 25 bis 30 bar verdichtet, wobei gleichzeitig eine Überhitzung auf etwa 220 bis 280° C auftritt, und dem Veresterungsreaktor 7 zugeführt. Der Sumpf des Ausdampfbehälters 5 wird zur weiteren Aufarbeitung in den Prozeß zurückgeführt. Am Sumpf des Reaktors 7 wird der Rohester abgezogen. Am Kopf des Reaktors 7 wird das Oxidat aufgegeben,und die methanol-haltigen Brüden fallen an.

Figur 2 zeigt ein Schema wie nach Figur 1 mit den besprochenen Prozeßströmen und zusätzlich die Anwendung einer Brüdenentspannungsturbine. Die Brüden aus dem Veresterungsreaktor 7 werden in dem Wäscher 8 bei beispielsweise 22 bis 27 bar und 175 bis 195°C mit Prozeßwasser oder auch dem Kondensat des Dephlegmators 9 gewaschen. Die am Kopf des Wäschers austretenden gereinigten Brüden werden direkt oder über einen Aufheizer 14 auf die Brüdenentspannungsturbine 10 mit einem Druck von etwa 21 bis 26 bar und einer Temperatur von 175 bis 250° C gegeben. In der Entspannungsturbine 10 erfolgt eine Entspannung der Brüden auf einen Druck von beispielsweise 0,1 bis 8 bar. Die entspannten Brüden werden in das Verfahren zurückgeführt. Der in dem Wäscher 8 anfallende nebenprodukt-haltige Strom wird mit der Pumpe 13 in den Veresterungsreaktor 7 zurückgeführt.

**Patentansprüche**

1. Verfahren zur Herstellung von Terephthalsäuredimethylester (DMT) aus p-Xylol (p-X) und Methanol durch Oxidation in flüssiger Phase mit Luftsauerstoff in Gegenwart von gelösten Schwermetallverbindungen als Katalysator eines Gemisches aus p-X und einer überwiegend p-Toluylsäuremethylester (p-TE) enthaltenden in die Oxidation zurückgeführten Fraktion zu einem hauptsächlich p-Toluylsäure (p-TS) und Terephthalsäuremonomethylester (MMT) enthaltenden Oxidat bei einer Temperatur von 140 bis 170° C und einem druck von 4 bis 8 bar, Veresterung des Oxidats mit auf erhöhten Druck gebrachtem flüssigem und anschließend verdampftem Methanol bei einer Temperatur von 220 bis 280° C und einem Druck von 20 bis 30 bar zu einem hauptsächlich p-TE und DMT enthaltenden Rohester, Abzug der Rohesterfraktion und einer methanol-haltigen Brüdenfraktion aus der Veresterung, destillative Auftrennung des Rohesters in eine p-TE-Fraktion, die in die Oxidation zurückgeführt wird eine Roh-DMT-Fraktion, die einer weiteren Aufarbeitung durch Umkristallisation in Methanol unterzogen wird,und eine Rückstandsfraktion, die gegebenenfalls einer thermischen Nachbehandlung oder einer reaktiven Behandlung mit Methanol unterzogen und nach anschließender destillativer Aufarbeitung teilweise in das Verfahren zurückgeführt wird, dadurch gekennzeichnet, daß man die Veresterung des Oxidats mit einem durch Kompression auf den Veresterungsdruck verdichteten, durch die Kompression auf eine Endtemperatur von 150 bis 300° C gebrachten methanol-haltigen Dampf durchführt, wobei das Verdichtungsverhältnis des der Kompression unterworfenen methanol-haltigen Dampfes 1,2:1 bis 15:1 beträgt und dieser methanol-haltige Dampf aus dem Filtrat der Umkristallisation der Roh-DMT-Fraktion durch Verdampfung oder auch durch Rektifikation der methanol-haltigen Brüdenfraktion und gegebenenfalls zusätzlicher methanol-haltiger Prozeßströme bei einem Druck von 2 bis 20 bar gewonnen wird.

2. Verfahren nach Anspruch 1, dadurch gekennzeichnet, daß der der Kompression unterworfene methanol-haltig Dampf durch Eindampfung oder auch Rektifikation bei einem Druck von 4 bis 8 bar erzeugt wird.

3. Verfahren nach Anspruch 1, dadurch gekennzeichnet, daß das Verdichtungsverhältnis des der Kompression unterworfenen methanol-haltigen Dampfes 3:7 bis 9:1 und die bei der Kompression erreichte Endtemperatur am Verdichteraustritt 220 bis 280° C betragen.

4. Verfahren nach Anspruch 1, dadurch gekennzeichnet, daß die aus der Veresterung abgezogenen Brüden gewaschen und anschließend in einer Brüden-Entspannungsturbine zum Antrieb eines Methanolkompressors oder eines Generators entspannt und anschließend in das Verfahren zurückgeführt werden.

5. Verfahren nach Anspruch 4, dadurch gekennzeichnet, daß die Brüden vor Eintritt in die Turbine teilweise kondensiert werden und ein Teil des Kondensats für die Brüdenwaschung verwendet wird.

6. Verfahren nach Anspruch 4, dadurch gekennzeichnet, daß die Brüden mit Prozeßwasser gewaschen werden.


**Claims**

1. Process for the production of terephthalic acid dimethyl ester (DMT) from p-xylene (p-X) and methanol by oxidation in the liquid phase with atmospheric oxygen in the presence of dissolved heavy metal compounds as catalyst at a temperature of 140 to 170° C and a pressure of 4 to 8 bar, of a mixture of p-X and a predominantly p-toluic acid methyl ester (p-TE) containing fraction led back into the oxidation to form a chiefly p-toluic acid (p-TS) and terephthalic acid monomethyl ester (MMT)-containing oxidation product, esterification of the oxidation product at a temperature of 220 to 280° C and a pressure of 20 to 30 bar, with liquid methanol brought to an elevated pressure and then evaporated, to form a chiefly p-TE and DMT containing crude ester, withdrawal of the crude ester fraction and a methanol-containing vapour fraction from the esterification, distillative separation of the crude ester into a p-TE fraction which is led back to the oxidation, a crude DMT fraction which is subjected to a further working up by recrystallisation in methanol and a residual fraction which is optionally subjected to a thermal after treatment or a reaction treatment with methanol, and, after subsequent distillative working up, is led back partially into the process, characterised in that the esterification of the oxidation product is carried out with a compressed methanol-containing vapour brought by the compression to a final temperature of 150 to 300° C, with the compression ratio of the methanol-containing vapour subjected to the compression amounting to 1.2:1 to 15:1 and with this methanol-containing vapour being recovered from the filtrate from the recrystallisation of the crude DMT-fraction by vaporisation or by rectification of the methanol-containing vapour fraction and optionally additional methanol-containing process streams at a pressure of 2 to 20 bar.

2. Process according to claim 1, characterised in that the compressed methanol-containing vapour is produced by evaporation or even rectification at a pressure of 4 to 8 bar.

3. Process according to claim 1, characterised in that the compression ratio of the methanol-containing vapour subjected to the compression amounts to 3:1 to 9:1 and the end temperature reached in the compression amounts, on emergence from the compressor, to 220 to 280° C.

4. Process according to claim 1, characterised in that the vapours withdrawn from the esterification are washed and then expanded in a vapour-expansion turbine for driving of a methanol compressor or of a generator and are then led back into the process.

5. Process according to claim 4, characterised in that the vapours are partially condensed before entry into the turbine and a part of the condensate is used for the washing of the vapours.

6. Process according to claim 4, characterised in that the vapours are washed with process water.


**Revendications**

1. Procédé de préparation de téréphtalate de diméthyle (DMT) a partir de p-xylene (p-X) et de méthanol par oxydation, en phase-liquide avec de l'oxygène de l'air en présence dé composés de métaux lourds dissous comme catalyseur, d'un mélange de p-X et d'une fraction recyclée dans la phase d'oxydation contenant essentiellement du p-toluate de méthyle (p-TE) pour obtenir un produit d'oxydation renfermant principalement de l'acide p-toluique (p-TS) et du téréphtalate monométhylique (MMT), à une température de 140 à 170° C et sous une pression de 4 à 8 bars, par estérification du produit d'oxydation avec du méthanol liquide amené à une pression accrue et aussitôt vaporisé à une température de 220 à 280° C et sous une pression de 20 à 30 bars en donnant un ester brut contenant principalement du p-TE et du DMT, par soutirage à partir de la phase d'estérification de la fraction d'ester brut et d'une fraction de vapeur contenant du méthanol, par séparation par distillation de l'ester brut en une fraction de p-TE qui est recyclée vers l'oxydation, en une fraction de DMT brut qui est soumise à un traitement ultérieur par recristallisation dans du méthanol et en une fraction résiduelle qui est, le cas échéant, soumise à un posttraitement thermique ou à un traitement réactif avec du méthanol et est en partie renvoyée dans le procédé après un traitement par distillation subséquent, caractérisé en ce qu'on effectue l'estérification du produit d'oxydation avec de la vapeur contenant du méthanol comprimée jusqu'à la pression d'estérification, amenée sous l'effet de la compression à une température finale de 150 à 300° C, moyennant quoi le taux de compression de la vapeur méthanolique soumise à la compression s'élève à 1,2:1 - 15:1 et cette vapeur méthanolique est récupérée par vaporisation à partir du filtrat de la recristallisation de la fraction de DMT brut ou également par rectification de la fraction de vapeur méthanolique et, éventuellement des courants de procédé méthanoliques additionnels, sous une pression de 2 a 20 bars.

2. Procédé selon la revendication 1, caractérisé en ce que la vapeur méthanolique soumise à la compression est produite par évaporation ou également par rectification sous une pression de 4 à 8 bars.

3. Procédé selon la revendication 1, caractérisé en ce que le taux de compression de la vapeur méthanolique soumise à la compression est de 3:1 à 9:1 et qu'au cours de la compression, la température finale atteinte à la sortie du compresseur s'élève à 220-280°C.

4. Procédé selon la revendication 1, caractérisé en ce que les vapeurs soutirées de la phase d'estérification sont lavées, consécutivement détendues dans une turbine de detente des vapeurs en vue de l'entraînement d'un compresseur de méthanol ou d'un générateur et sont ensuite recyclées dans le procédé.

5. Procédé selon la revendication 4, caractérisé en ce que les vapeurs sont partiellement condensées avant leur admission dans la turbine et qu'une partie du condensat est utilisée pour le lavage des vapeurs.

6. Procédé selon la revendication 4, caractérisé en ce que les vapeurs sont lavées avec de l'eau du procédé.

Brüden
zum Prozeß

Oxidat

Methanoldampf
zur Nachbehandlung
von Rückstand

Filtrat
vom Prozeß

el. Energie

ND vom
Prozeß

7

1

5

4

3

2

6

Rohester zum Prozeß

eingedicktes Filtrat zum Prozeß

Filtrat zum Prozeß

Fig. 1

0156133

Methanoldampf
zur Nachbehandlung
von Rückstand

Filtrat
vom Prozeß

ND vom
Prozeß

Prozeßwasser

K D

Oxidat

Bröden zum Prozeß

Rohester zum Prozeß

eingedicktes Filtrat zum Prozeß

Filtrat zum Prozeß

M/~G

Fig. 2

0156133